# NEW EUROPEAN PATENT SPECIFICATION

(11) **EP 0 784 490 B2**
(45) Date of publication and mention of the opposition decision: **11.08.2010**
(45) Mention of the grant of the patent: 18.08.2004
(21) Application number: 95935719.5
(22) Date of filing: 03.10.1995
(51) Int. Cl.: A61L 27/00, A61L 27/24, A61L 27/26, A61L 27/40

(54) **DIFFERENTIALLY BIODEGRADABLE BIOMEDICAL IMPLANTS**
DIFERENTIELL BIOABBAUBARE MEDIZINISCHE INPLANTATE
IMPLANTS BIOMEDICAUX PRESENTANT DES CARACTERISTIQUES DIFFERENTES DE BIODEGRADABILITE

(30) Priority: 03.10.1994 WO PCT/US94/11209
(43) Date of publication of application: 23.07.1997
(73) Proprietor: Angiotech BioMaterials Corp., Palo Alto, CA 94303 (US)
(72) Inventor: SIERRA, David, H., Atherton, CA 94027 (US)
(74) Representative: Donald, Jenny Susan
(86) International application number: PCT/US1995/012802
(87) International publication number: WO 1996/010428

(56) References cited:
- EP-A- 0 192 068
- EP-A- 0 539 751
- EP-A- 0 560 014
- WO-A-90/03768
- WO-A-95/03011
- A. Pandit & D. Feldman "The effect of a porous degradable fibrin scaffold on wound healing", abstract of a presentation given at the 20th Annual Meeting of the Society fro Biomaterials, 5 - 9 April 1994
- A Pandit and D. Feldman, "In Vivo Response of a porous fibrin scaffold on wound healing", abstract of a presentation given at the 4th Annual Scientific Meeting of the Wound Healing Society, San Francisco, 18 - 20 May 1994

## Description

This invention is in the general field of biomaterials. More specifically, the invention is directed to biomedical implants, their composition and methods of preparation and use.

Biomaterials have been used for implantation into the human body to act as supports for wound and solid tissue healing. Matrices useful for this purpose should have the ability to adhere and conform to the wound site and surrounding tissue. Ideally, they also should facilitate accumulation of fibroblasts, endothelial cells and wound healing regulatory cells to promote connective tissue deposition and angiogenesis.

EP-A-0 539 751 discloses a biodegradable polymer composition composed of a thermoplastic or thermosetting polymer which is capable of forming a biodegradable and/or bioerodible microporous, solid or gelatinous polymer matrix.

EP-A-0 560 014 discloses a biodegradable film dressing formed from a liquid composition of at least one biodegradable/bioerodible thermoplastic polymer in a pharmaceutically acceptable solvent.

U.S. Patent No. 4,849,285 to Dillon is directed to a composite, self-supporting agglomerated macrostructure useful as a surgical implant. The macrostructure is a matrix of polytetrafluoroethylene resin and cured silicone that has uniformly distributed within it a particulate material. These particulates have a maximum size of about 2000 microns and may be hydroxyapatite or tricalcium phosphate. This particular macrostructure, therefore, is a conrposite of ceramic particulate material and organic biomaterials that is uniformly permeated by a network of open pores. The pores are formed by incorporating sodium chloride into the composite and thereafter leaching it out in the manufacturing process.

U.S. Patent No. 4,843,112 to Gerhart et al. is directed to a bone cement composed of a particulate biocompatible calcium phosphate ceramic and a resprbable calcium salt disperses in a crosslinked biodegradable polyester matrix. Pores are created in the matrix by body fluids creating small voids or cavities in the polymer matrix.

U.S. Patent No. 5,141,522 to Landi et al. describes a composite of two or more biocompatible polymers useful for mammalian tissue repair. One of the polymers is polytetrafluoroethylene (PTFE), which is the reinforcing binder. A bioabsorbable component that may be a lactone carbonate or a lactide is contained within the structure of the PTFE and serves to enhance ingrowth of tissue.

Additional disclosures of PFTE compositions useful as implants include, but are not limited to, U.S. Patent Nos. 5,141,522; 5,098,779; and 4,863,974. The PFTE component of these compositions serves as a nonabsorbable microfibrillar structural support. A bioabsorbable component is contained or coated on the structural support. The PFTE is polymerized prior to implantation of the compositions.

U.S. Patent No. 4,373,217 to Draenert is directed to a polymeric implant material that has an acrylate, polymethacrylate or copolymer base with dispersed resorbable tricalcium phosphate of 50 to 300 microns with an available pore volume of less than 0.1 mL/g. This particular material is said to allow for a firm bond between implant and body tissue. Resorption of tricalcium phosphate particles at the surface of the implant are resorbed into the body to promote bone growth in the marginal porosity produced. In order to ensure absorption of liquid monomer into the porous calcium phosphate, a filler that is also resorbable in the body is included to fill the pore volumes of the calcium phosphate.

U.S. Patent No. 4,898,734 to Mathiowitz et al. also involves a precast solid polymeric implant material. A continuous polymeric matrix made of, for example, polyurethane or polystyrene, is embedded with microcapsules or microspheres that may contain material for subsequent release. The spheres may be removed from the matrix by bioerosion. For creation of a vascular graft, erodible microspheres are entrapped within a tube-shaped slower-degrading polymer matrix. Rapid erosion of the spheres results in pores for cell seeding and vascularization with the matrix providing support until there is sufficient cell growth to create structural integrity.

U.S. Patent No. 4,950,483 to Ksander et al. describes a collagen implant useful for wound healing. The implant is made of collagen and has a bulk density of 0.01 to 0.03 g/cm³ and is said to have a pore size sufficient to permit cell ingrowth. Bioactive agents such as FGF and TGF-β may be incorporated into the implant.

U.S. Patent No. 5,077,049 to Dunn et al. is directed to a method for restoring periodontal tissue. A biodegradable liquid polymeric system designed to generate a porous structure when cured into a barrier membrane is administered to the soft-tissue defect. The pores will form as a result or water-soluble material included in the liquid material. The liquid material injected into the defect provides a scaffold that is filled with new bone cells that gradually replace the water-soluble polymer.

U. S. Patent No. 4,902,295 to Walthall et al. involves a transplantable artificial tissue. The tissue is made by mixing a polymerizing matrix with reversible gel precursors in an aqueous solution with viable cells. The gel, which may be alginate, a gum or agarose, is then dissolved to provide a porous matrix for implantation.

None of the above-described references describes a biomedical implant material with a differentially degradable matrix and porosifying agent where polymerization occurs in *situ* and is made of a biopolymeric material.

Thus, the invention provides an in situ polymerizable implant composition capable of forming a differentially biodegradable biomedical implant, useful for implantation into a patient, the implant comprising:
a biodegradable biocompatible matrix material being one or more of collagen, fibrin, fibrinogen, gelatin and mixtures, combinations and derivatives thereof; and
a biodegradable biocompatible porosifying agent in the form of a particulate material; wherein the porosifying agent is gelatin, gelatinized collagen, collagen, fibrin, fibrinogen, a protein, degradable polyester, liposome and alginate, or a lipid with an emulsifier;
the matrix material having a slower degradation rate than that of the porosifying agent;
wherein polymerization and solidification of the implant occur in situ and wherein, upon degradation of the porosifying agent, a continuous porous network is formed within the implant.

### Modes of Carrying Out the Invention

### Definitions

As used herein, certain terms will be used which have defined meanings.

By "biodegradable" or "bioerodible" as it relates to the porosifying agent is intended a material that will dissolve In situ as a result of exposure to an aqueous environment in less than a week, preferably between about 1 and 72 hours, more preferably between about 2 and 12 hours. Dissolution may occur as a result of a number of different mechanisms such as simple diffusion, hydrolysis, enzymatic cleavage, ion exchange, autocatalysis, osmosis, degradation, free-radical cleavage, radiation effect, thermal melting, and chemical dissolution. Hydrolysis is the preferred mechanism for biodegradation. As such, the biodegradation of the porosifying material is distinguishable from prior art "leaching" of water-soluble drugs and salts, such as particulate calcium salts, e.g., tricalcium phosphate. Typically, these water-soluble drugs or salts merely create small voids or cavities on the surface of the matrix in contrast to the porous network provided by the biodegradable porosifying agents described herein.

By "slowly biodegradable" or "slowly bioerodible" as it relates to the matrix material is intended a material that will not dissolve *in situ* (or in an aqueous environment). within a week, or may dissolve in a period of from about one week to 24 months, preferably a period of between about 1 to 12 months. It also is intended to exclude material such as a polyether that is only degradable outside the range of normal body temperature and in organic solvents. Examples of this type of excluded polyether include low molecular weight aliphatic polyethers which are soluble in aqueous solutions of methanol, ethanol or acetone.

The term "porosifying-agent" intends particulate materials that include but are not limited to materials in the form of solid or hollow spheres, extruded rods, or other convenient shapes. Typically, the particulate has a mean diameter of between about 10 and 500 µm, more typically between about 20 and 200 µm. The particles are generally spherical in shape but other shapes such as rhombic, irregular, stellate and other crystalline type shapes may be used. The agents are present in a concentration of at least about 12% per volume of the matrix material, preferably the concentration is between about 12 and 99% per volume of the matrix material, more preferably between about 20 and 90% per volume of the matrix material such that as the agent biodegrades a continuous porous network or pathway is formed within the implant. In one embodiment, components such as calcium salts, alginate, gum or agarose are specifically excluded.

The term "matrix" intends the portion of the implant material that acts as the support network; it is the slower biodegrading portion of the implant.

The term "continuous porous network" is intended to describe a network of micro-spacings or an internal micro-network formed by the biodegradation of the porosifying agent. The micropores are internally and externally interconnected to form a tunnel-like system or network within and throughout the matrix.

### The Implant Material

This invention is directed to a biomedical implant comprising a non-toxic, slowly biodegradable biomedical matrix material and a non-toxic, biodegradable material that acts as a porosifying agent. The porosifying agent is present in sufficient quantity and particulate size to result in a continuous, porous network within the matrix once it has degraded.

The implant is biocompatible and is capable of solidifying and polymerizing *in situ*. Further, the matrix is slowly biodegradable as defined above and made from a material with a slower degradation rate than the porosifying agent. Degradation (or dissolution) rates of particular substances in water are generally available information.

The matrix material is one or more of fibrin, fibrinogen, gelatin, including analogs (e.g., fibrinogen analog as PCT WO 94/16085 to Irani for "Hybrid proteins having crosslinking and tissue binding activities", mixtures, combinations and derivatives (e.g., methylated collagen of the above. Preferred mixtures of the above for the matrix is a fibrin/collagen matrix in combination with gelatin as the porosifying agent. In one embodiment, polytetrafluoroethylene (PTFE), calcium phosphate ceramics, and materials that are not amenable to polymerization *in situ* such as polyethylene are specifically excluded as matrix materials.

The porosifying agent is biocompatible'and biodegradable as described above. The porosifying agents include gelatin, gelatinized collagen, collagen, fibrin, fibrinogen, proteins in solid state like albumin powder, degradable polyesters (polylactic or polyglycolic acid), liposomes and alginates, analogs, mixtures, combinations and derivatives of the above. Preferred mixtures of porosifying agents include pegulated particulates, albumin microspheres and gelatin. The porosifying agents may be in a solid state, such that they dissolve over a period of time or they may be altered such that they are in a sparingly soluble state. This may be accomplished, for example, by altering the pI, for example by methylation or succinylation or by conjugating the porosifying agent to polyethylene glycol (MW 1 to 50 Kd) or by crosslinking the porosifying agent with glutaraldehyde.

The matrix material is biodegradable but at a rate which is slower than the porosifying agent. As such, materials such as PTFE and bone substi tutes as described above are specifically excluded in that these materials are nonabsorbable yet biocompatible materials. Additionally, PTFE is not capable of polymerization *in situ.* When the matrix is composed of collagen, the collagen is preferably not chemically crosslinked, although it can be if desired.

In addition to the matrix material and porosifying agent, the implants may further include growth factors, including, but not limited to, epidermal growth factor (EGF), transforming growth factor β (TGFβ-1, TGFβ-2), platelet derived growth factor (PDGF-AA, PDGF-AB, PDGF-BB), fibroblast growth factor (FGF), insulin-like growth factors (IGF), tumor necrosis factors (TNF), colony stimulating factors (CSFs), nerve growth factors (NGF), brain-derived neurotropic factor (SDNF-(Amgen, Thousand Oaks, CA and Regeneron, Inc. Tarrytown, NY), ciliary neurotropic factor (CNTF) (Amgen, Thousand Oaks CA and Regeneron, Inc. Tarrytown, NY) and the like, and/or therapeutic agents including but not limited to cytokines, interleukins (Il-1, IL-2) or other co-factors such as heparin or calmodulin, antibiotics, antineoplastic and antibacterials, to further stimulate or control tissue remodeling, or to control sepsis. These agents can be incorporated into the matrix material, the porosifying agent, or both. When the therapeutic is incorporated into the porosifying agent, it is released at a rate greater than the matrix material.

An important characteristic of the implant is that the porosifying agent degrades faster than the matrix material. For example; if fibrin is used as the matrix, then gelatin, which degrade more rapidly in water (and thus *in situ*) than does fibrin, may be used as the porosifying agent. However, if fibrin is used as the porosifying agent, then collagen may be used as the matrix since it degrades more slowly than does fibrin.

### Preparation of the Implant

### in situ Polymerizing Systems

In an *in situ* polymerization system, the porosifying agent may be mixed as a dry phase with the matrix which may be in a semi-solid, liquid or dry particulate phase. An appropriate catalyst or co-factor may be added to the mixture, or the porosifying agent itself may contain such catalyst or co-factor that will initiate polymerization.

Fibrin sealants are an example of an *in situ* polymerizing system. Fibrin sealants are two component tissue adhesive systems that are in a relatively viscous liquid form until both components are mixed together and polymerize at the surgical application site into a relatively dense gel. Thrombin in combination with Ca²⁺ catalyzes the polymerization of fibrinogen, converting the fibrinogen into fibrin polymer. Further, thrombin and Ca²⁺ activate coagulation Factor XIII, which effects covalent crosslinking of fibrin. The rate of proteolytic degradation of the fibrin polymer clot is decreased and mechanical stability is increased as a result of the covalent crosslinking of the polymer.

The fibrin polymer clot is porous, but only at a range of 1 to 5 microns in mean diameter, too small to permit cellular ingrowth. Accordingly, macrophage activity is sustained over periods of time longer than optimal for degradation and remodeling, and the fibrin polymer clot acts as a barrier until phagocytosis is complete. Where a porosifying,agent is added according to the present invention, tissue reunion is improved as a result of the continuous pathway formed in the clot when the porosifying agent degrades *in situ.*

For systems where the matrix is made of fibrin, particulates may be incorporated directly into the fibrinogen component which is obtained in lyophilized form. The particulates may be alginate, gelatin, polylactic acid/polyglycolic acid (PLA/PGA) hollow spheres, lipid in an emulsifier system (e.g., lecithin, Triton, lauryl sulfate, or Tween-80). hyaluronic acid and liposomes or other materials that degrade at a rate faster than the fibrin matrix and will create a continuous porous network once degraded. The porosifiers may be incorporated in dry or liquid or semisolid form. Alternatively, the porosifier may be mixed just prior to, or during, application of the system to the repair site. In another embodiment, collagen can be used as the matrix material of the inventive implants. When collagen is the matrix material, the porosifying agent may preferably contain an effective fibrin-forming-amount of thromboplastin (Ortho Diagnostic, Raritan, N.J). These composites are particularly useful for tissue repair or effecting hemostasis by administering to the wound or treatment site a therapeutic amount of the composite. The particulate is preferably a hydrophilic porosifier such as gelatin, gelatinized collagen, fibrin or a salt.

### Use of the Implant

When the implant is placed or applied to a desired site in vivo, the porosifying agent biodegrades relatively rapidly, thus leaving behind an interconnecting network of pores to permit tissue and fluid influx into the matrix. The matrix then acts as a scaffolding for the migrating cells (e.g. macrophages, fibroblasts, and neovascular endothelial cells) and will degrade as these cells express connective tissue components for remodeling and regeneration.

The use of a matrix with a component that degrades *in situ* imparts several advantages over conventional porous implant configurations. First, porous implants tend to shrink in volume due to pressure from surrounding tissue, thus minimizing the benefits of controlled pore size and minimizing the amount of tissue ingrowth that can take place. Where a porosifying agent that degrades *in situ* is added, however, the cells involved in wound healing migrate into the network and minimize shrinkage of the implant.

A further benefit of an *in situ* degrading porosifying agent is that the porosifying agent acts as a mechanical stabilizer, permitting the formation of a porous network within the matrix. Materials such as gelatin, especially crosslinked gelatin, fibrin are especially useful, as the porosifying agent. Crosslinking may be accomplished by the addition of agents such as SPEG (polyethylene glycol succinimydyl), glutaraldehyde or diisocyonate, or dehydrothermally.Where calcium alginate is the porosifying agent, the guluronic/mannuronic acid segment ratio may be optimized for *in vivo* dissolution over the targeted period of time. Where fibrin is the porosifying agent, a high quantity of plasmin (≥0.2 mg/mL) is also useful, permitting a degradation rate proportional to the quantity of plasminogen present.

Another benefit derived from using an *in situ* biodegradable porosifying agent is that the mechanical properties of the implant both pre- and post-polymerization can be altered, tailoring the viscosity of the applied material and improving its mechanical stability *in situ.* The porosifying agent increases the stiffness modulus of the implant while it is still relatively undissolved. As dissolution occurs the contribution to the modulus by the porosifying agent decreases. Deposited ground substances (i.e. mucopolysaccharides, glycosaminoglycans, nectins and other proteoglycans) and collagen and inflammatory cells are exchanged, thus the overall modulus status remains roughly the same throughout the life-span of the matrix.

The rate of degradation of the implant materials will vary depending upon the material used (crosslinked gelatin the slowest) as well as the relative vascularity of the application site (liver, the fastest, subcutaneous, the slowest). A fibrin matrix will last usually from 5 to 14 days, depending upon concentration, plasminogen content and anatomic region. Higher fibrin and lower plasmin concentrations will decrease degradation rates. The addition of antiproteases such as ε-amino-n-caproic acid or aprotinin will retard degradation further. Once the implant is applied to the wound site, the porosifying agent begins to dissolve. The resultant porosity permits firm anchoring to the wound bed by host fibrin clots intercalating. through the porous network. Leucocytes, macrophages, lymphocytes and fibroblasts then migrate through the pores, breaking down the fibrin implant matrix and initiating deposition of ground tissue substances (e.g. proteoglycans) and collagen. By way of example, implants tailored to last for 7 to 14 days may be applied to donor graft beds, chronic decubitus ulcers, resected tumor sites or bone tissue gaps.

*In situ* polymerizing systems are introduced into the repair site by a variety of means. They may be poured onto the site directly or by a dispenser which permits control of the amount of material in the system, as well as the area covered. The implants may be used as occlusive or fluid-tight dressings or sealants in anatomic region where it would be difficult to use a precast dressing, such as in endoscopic procedures. An example of a dispensing device is the DUPLOJECT® fibrin sealant delivery device (Immuno AG, Vienna, Austria).

It is apparent to those skilled in the art that the compositions described herein are useful for the preparation of medicaments for any suitable use, for example, tissue repair or for the release of therapeutic agents.

### Examples

### Comparative Example 1 - Preparation of an in situ Polymerizing Fibrin Implant

In a tuberculin syringe with a 20 gauge needle, concentrated fibrinogen-Factor XIII (60mg/mL) in tris-buffered saline (pH 7.2) is mixed with polyethylene glycol particulate (10000, MW, mean diameter 150 µm) to 50 vol%.

### Comparative Example 2 - Preparation of an in situ Polymerizing Calcium Alginate Implant

Calcium alginate microspheres (mean diameter 100 µm) prepared as described in Gospodarowicz and Cheng J. Cell Physiol 128:475-484 (1936).

These are added in a syringe to 50 vol % as described in Example 1.

### Example 3 - Preparation of an in situ Polymerizing Gelatin Implant

### SPEG-crosslinked gelatin

5 mL of concentrated collagen slurry in phosphate buffered saline (pH 7.2, 35 mg/m/mL, Zyderm I , Collagen Corp, Palo Alto, CA) is heated to 60°C for 1 hour in a water bath, and then chilled to 37°C to produce gelatin. Phosphate-buffered saline is. added to dilute the gelatin concentration to 15 mg/mL. Sufficient SPEG is added to the gelatin solution for a final concentration of 10 mg/mL. The gelatin-SPEG solution is allowed to cool to room temperature and gel. The gel is lyophilized and pulverized by a grinding mill. The powder is sieved and particles in the range of 20 to 150 µm mean diameter are saved and sterilized by electron beam irradiation (2.5 Mrad dose).

### The Matrix

The lyophilized SPEG crosslinked gelatin particulate is mixed with lyophilized fibrinogen-Factor XIII in a 1:1v/v ratio. The powdered mixture is loaded into a dual plunger syringe system, containing both the lyophilate and the reconstituting buffer, Tris-buffered saline (TBS).To reconstitute the gelatin-fibrinogen mixture, the plunger is depressed, forcing the diluent into the chamber containing the lyophilate. After several minutes incubation, the resultant slurry is ready to use.

## Claims

1. An *in situ* polymerizable implant composition capable of forming a differentially biodegradable biomedical implant, useful for implantation into a patient, the implant comprising:
a biodegradable biocompatible matrix material being one or more of collagen, fibrin, fibrinogen, gelatin and mixtures, combinations and derivatives thereof; and
a biodegradable biocompatible porosifying agent in the form of a particulate material, wherein the porosifying agent is gelatin, gelatinized collagen, collagen, fibrin, fibrinogen, a protein, degradable polyester, liposome and alginate, or a lipid with an emulsifier;
the matrix material having a slower degradation rate than that of the porosifying agent;
wherein polymerization and solidification of the implant occur *in situ* and wherein, upon degradation of the porosifying agent, a continuous porous network is formed within the implant.

2. A composition according to claim 1 further comprising a growth factor.

3. A composition according to claim 2 wherein the growth factor is TFGβ-1, TGFβ-2, FGF, EGF, PDGF-AA, PDGF-AB, PDGF-BB, IGF, TNF, CSF, BDNF, CNTF, NGF or an analog, mixture or derivative thereof.

4. A composition according to any preceding claim further comprising a therapeutic agent.

5. A composition according to claim 4 wherein the therapeutic agent is a cytokine, interleukin, heparin, calmodulin, thromboplastin, antibiotic, antineoplastic and/or an antibacterial.

6. A composition as defined in any preceding claim for use in a method of treatment.

7. The use of a composition as defined in any of claims 1 to 6 for the manufacture of a medicament for repair of tissue.

8. The use according to claim 7 which is for tissue hemostasis.

9. The use according to claim 7 or 8 for the *in situ* delivery of a chemotherapeutic or therapeutic agent.

## Patentansprüche

1. *In-situ* polymerisierbare Implantat-Zusammensetzung, die zur Bildung eines unterschiedlich bioabbaubaren, biomedizinischen Implantats imstande ist, welches für die Implantierung bei einem Patienten nützlich ist, wobei das Implantat Folgendes umfasst:
ein bioabbaubares, biokompatibles Matrixmaterial, das eines oder mehrere aus Kollagen, Fibrin, Fibrinogen, Gelatine und Mischungen, Kombinationen und Derivaten davon ist; und
ein bioabbaubares, biokompatibles, porös machendes Mittel in der Form eines teilchenförmigen Materials, wobei das porös machende Mittel Gelatine, geliertes Kollagen, Kollagen, Fibrin, Fibrinogen, ein Protein, abbaubarer Polyester, Liposom und Alginat oder ein Lipid mit einem Emulgator ist;
wobei das Matrixmaterial eine langsamere Abbaurate aufweist als das porös machende Mittel;
wobei die Polymerisation und Verfestigung des Implantats *in situ* erfolgt und wobei beim Abbau des porös machenden Mittels ein kontinuierliches poröses Netzwerk innerhalb des Implantats gebildet wird.

2. Zusammensetzung nach Anspruch 1, weiterhin umfassend einen Wachstumsfaktor.

3. Zusammensetzung nach Anspruch 2, wobei der Wachstumsfaktor TFGβ-1, TGFβ-2, FGF, EGF, PDGF-AA, PDGF-AB, PDGF-BB, IGF, TNF, CSF, BDNF, CNTF, NGF oder ein Analogum, eine Mischung oder ein Derivat davon ist.

4. Zusammensetzung nach irgendeinem vorhergehenden Anspruch, weiterhin umfassend ein therapeutisches Mittel.

5. Zusammensetzung nach Anspruch 4, wobei das therapeutische Mittel ein Cytokin, Interleukin, Heparin, Calmodulin, Thromboplastin, Antibiotikum, Antineoplastikum und/oder ein antibakterielles Mittel ist.

6. Zusammensetzung, wie in irgendeinem vorhergehenden Anspruch definiert, für die Verwendung in einem Behandlungsverfahren.

7. Verwendung einer Zusammensetzung, wie in irgendeinem der Ansprüche 1 bis 6 definiert, für die Herstellung eines Medikaments für die Reparatur bzw. Wiederherstellung von Gewebe.

8. Verwendung nach Anspruch 7, welche der Gewebe-Hämostase dient.

9. Verwendung nach Anspruch 7 oder 8 für die *in-situ*-Abgabe eines Chemotherapeutikums oder therapeutischen Mittels.

## Revendications

1. Une composition implant polymérisable *in situ* capable de former différentiellement un implant biomédical biodégradable, utile pour l'implantation chez un patient, l'implant comprenant :
une molécule matricielle biocompatible biodégradable étant une ou plusieurs des molécules de collagène, fibrine, fibrinogène, gélatine et les mélanges, combinaisons et dérivés de ceux-ci ; et
un agent rendant poreux biocompatible biodégradable sous forme d'une molécule de particules en suspension, dans lequel l'agent rendant poreux est la gélatine, le collagène gélatiné, le collagène, la fibrine, le fibrinogène, une protéine, un polyester dégradable, un liposome et un alginate, ou un lipide avec un émulsifiant ;
la molécule matricielle ayant un taux de dégradation plus lent que celui de l'agent rendant poreux ;
dans lequel la polymérisation et la solidification de l'implant se produit *in situ* et dans lequel, à la dégradation de l'agent rendant poreux, un réseau poreux continu se forme dans l'implant.

2. Une composition conforme à la revendication 1 comprenant en plus un facteur de croissance.

3. Une composition conforme à la revendication 2 dans laquelle le facteur de croissance est de TFGβ-1, TGFβ-2, FGF, EGF, PDGF-AA, PDGF-AB, PDGF-BB, IGF, TNF, CSF, BDNF, CNTF, NGF ou un analogue, mélange ou dérivé de ceux-ci.

4. Une composition conforme à l'une quelconque des revendications précédentes comprenant en plus un agent thérapeutique.

5. Une composition conforme à la revendication 4 dans laquelle l'agent thérapeutique est une cytokine, une interleukine, une héparine, une calmoduline, une thromboplastine, un antibiotique, un antinéoplasique et/ou un antibactérien.

6. Une composition conforme à l'une quelconque des revendications précédentes pour l'utilisation dans une méthode de traitement.

7. L'utilisation d'une composition comme définie dans l'une quelconque des revendications 1 à 6 pour la fabrication d'un médicament pour la réparation des tissus.

8. L'utilisation conforme à la revendication 7 qui est l'hémostase des tissus.

9. L'utilisation conforme à la revendication 7 ou 8 pour le libération *in situ* d'un agent chimiothérapeutique ou thérapeutique.
